# EUROPEAN PATENT APPLICATION

(11) **EP 2 244 026 A1**
(43) Date of publication of application: **27.10.2010**
(21) Application number: 09702057.2
(22) Date of filing: 05.01.2009
(51) Int. Cl.: F24F 6/12, A61L 9/14, B05B 17/06, C02F 1/46, F24F 6/00

(54) **ATOMIZER**

(30) Priority: 16.01.2008 JP 2008006925
(71) Applicant: SANYO Electric Co., Ltd., Moriguchi-shi Osaka 570-8677 (JP)
(72) Inventor: TANIDA, Yosuke, Osaka 531-0063 (JP); TANAKA, Koji, Osaka 573-0026 (JP)
(74) Representative: Glawe, Delfs, Moll
(86) International application number: PCT/JP2009/050165
(87) International publication number: WO 2009/090909

(57) **Abstract**

An atomizing apparatus (10) includes a housing (12), and a tank (40) accumulates tap water inside the housing. By electrolyzing the tap water by the electrolysis electrodes (120p, 120m), the water in the tank becomes electrolyzed water. At the first operation after a shortage of water state in the tank is detected, an initial electrolysis operating time is set to 20 minutes (step S210). In a case of not an operation after the shortage of water state but a restart of a normal operation, the initial electrolysis operating time is set to 5 minutes (step S224).

## Description

### TECHNICAL FIELD

The present invention relates to an atomizing apparatus. More specifically, the present invention relates to an apparatus which electrolyzes water including chlorine ion, such as tap water to generate electrolyzed water, and emit or diffuse mist of the electrolyzed water.

### PRIOR ART

One example of an apparatus emitting mist of electrolyzed water of such a kind is disclosed in Japanese Patent Application Laying-Open No. 2007-202753 [A61L 9/16 C02F 1/46] (Patent Document 1).

The related art of the Patent Document 1 is an air disinfecting apparatus, but has a common technique to an atomizing apparatus in that it emits or diffuses mist of electrolyzed water. In the related art, an AC power source is normally connected, and even if the power switch is turned off, an electrolyzed water generation time is controlled by the power supplied by a dark current. Furthermore, when water in a tank is run out, this is notified to a computer by a managing means, such as a current value flowing through a float electrode or a direct input with a switch, and the computer resets a timer managing an electrolyzed water generation time to newly manage the electrolyzed water generation time. Thus, it is possible to securely manage a concentration of the electrolyzed water.

As in the related art, in order to properly maintain the electrolyzed water concentration, the power has to be constantly supplied. On the other hand, in order to make such an atomizing apparatus and a disinfecting apparatus portable, they are powered from a battery. In the battery-powered apparatus, considering power consumption, the power cannot be supplied constantly, making it difficult to control the electrolyzed water concentration.

### SUMMARY OF THE INVENTION

Therefore, it is a primary object of the present invention to provide a novel atomizing apparatus.

Another object of the present invention is to provide an atomizing apparatus capable of managing an electrolyzed water concentration without increasing power consumption.

The present invention employs following features in order to solve the above-described problems. It should be noted that reference numerals and the supplements inside the parentheses show one example of a corresponding relationship with the embodiments described later for easy understanding of the present invention, and do not limit the present invention.

A first invention is an atomizing apparatus comprising a water storage which stores water including chlorine ion; an electrolyzer which electrolyzes the water stored in the water storage by a pair of electrolysis electrodes into electrolyzed water; a mist generator which generates mist of the electrolyzed water in the water storage; a diffuser which diffuses the mist generated by the mist generator; a shortage of water detector which detects a shortage of water state when the water in the water storage is below a predetermined amount; a memory which memorizes the shortage of water state when the shortage of water state is detected by the shortage of water detector; a determiner which determines whether or not the shortage of water state is memorized by the memory at a start of an operation; and an initial operation time controller which sets a time of an initial electrolyzing operation by the electrolyzer to a different time depending on a determination result by the determiner.

In the first invention, an atomizing apparatus (10: reference numeral designating a corresponding portion in the embodiments. The same is true for the following description.) has a water storage like a tank (40), and an electrolyzer performs electrolysis by applying a voltage to electrolysis electrodes (120p, 120m) within the tank. A mist generator includes an ultrasonic wave vibrator (76), for example, and is supplied with the electrolyzed water from the tank, and generates and diffuses mist of the electrolyzed water.

A shortage of water detector (132, 124) detects a shortage of water state when the water of the water storage (40) is below a predetermined amount, specifically on the basis of the fact that a value of the current flowing to the shortage of water detecting electrode (124) is equal to or less than a threshold value. The computer (132) causes a shortage of water state memory like a shortage of water flag, for example, to store a shortage of water state when the shortage of water state is detected.

At a start of an operation, the computer (132) determines whether or not the shortage of water state is memorized with reference to the shortage of water state memory. Depending on whether the shortage of water state is stored or not, an initial electrolyzing operation time controller (132, S210, S218, S224, S228) changes the time of the initial electrolyzing operation by the electrolyzer.

According to the first invention, the time of the initial electrolyzing operation is controlled only at the start of the operation, so that it is possible to control a concentration of the electrolyzed water without increasing power consumption.

A second invention is an atomizing apparatus according to the first invention, wherein a first time is set for the initial electrolyzing operation when the determiner determines that the shortage of water state is memorized by the memory, and a second time shorter than the first time is set when the determiner does not determine that the shortage of water state is stored in the memory.

In the second invention, when the shortage of water state is stored, it is determined that it is shortly after the replenishment of tap water, for example, and thus the electrolyzed water concentration is approximately close to zero, so that by executing the initial electrolyzing operation for a relatively long time, for example, for a first time like 20 minutes, the electrolyzed water concentration is rapidly heightened. When the shortage of water state is not stored, it is determined that a normal operation is restarted, for example, so that the electrolyzed water concentration is high to a certain extent, and thus, the initial electrolyzing operation for a relatively short time, for example, for a second time like 5 minutes is executed.

According to the second invention, it is possible to satisfy reduction of the power consumption and retention of the electrolyzed water concentration.

A third invention is an atomizing apparatus according to the second invention, and further comprises a time storage which stores a remaining time of the initial electrolyzing operation when stop of the operation is instructed during the initial electrolyzing operation, wherein the initial operation time controller sets the remaining time as the initial electrolyzing operation time.

In the third invention, when the initial electrolyzing operation is interrupted, a remaining time with respect to the first time or the second time is stored in a remaining time register, for example, and when the operation is restarted, the initial electrolyzing operation is continued by the remaining time, so that it is possible to surely maintain the electrolyzed water concentration.

A fourth invention is an atomizing apparatus according to any one of the first to third inventions, and the shortage of water detector includes another shortage of water detecting electrode other than the pair of electrolysis electrodes, and detects whether the shortage of water state or not depending on whether or not a predetermined amount of current or more flows through the shortage of water detecting electrode during the initial electrolyzing operation.

In the fourth invention, the shortage of water detector detects whether a shortage of water state or not depending on whether a current flows into a shortage of water detecting electrode (124), but at this time also, a voltage has to be applied to the electrolysis electrode (120p). In the initial electrolyzing operation, a voltage has to be applied to the electrolysis electrode, and therefore, if the shortage of water detection is also executed at the same time, the electric power is not consumed for only the shortage of water detection, capable of efficiently performing reduction of the power consumption.

According to the present invention, it is possible to obtain the atomizing apparatus capable of managing the electrolyzed water concentration without increasing the power consumption.

The above described objects and other objects, features, aspects and advantages of the present invention will become more apparent from the following detailed description of the present invention when taken in conjunction with the accompanying drawings.

### BRIEF DESCRIPTION OF THE DRAWINGS

Figure 1 is an illustrative view showing an appearance of one embodiment of the present invention, Figure 1 (A) is a font view, and Figure 1 (B) is a plan view.
Figure 2 is an illustrative front view with a housing removed in order to show an internal configuration of Figure 1 embodiment, and partially broken away so as to pass through the center of a releasing opening shown in Figure 1 (B).
Figure 3 is an illustrative view showing a vibrator portion of Figure 1 embodiment in detail.
Figure 4 is an illustrative view of a fixing plate provided at an upper end of the housing shown in Figure 1 embodiment as seen from above.
Figure 5 is an illustrative view showing a lower surface plate of a lid placed above the fixing plate in Figure 1 embodiment.
Figure 6 is an illustrative view showing a different positional relation between the fixing plate and the lower surface plate (lid) in Figure 1 embodiment.
Figure 7 is an illustrative view showing an electrode unit in Figure 1 embodiment.
Figure 8 is a side view showing the electrode unit shown in Figure 7.
Figure 9 is an illustrative view showing a part of an air passage of Figure 1 embodiment in detail.
Figure 10 is a block diagram showing a configuration of Figure 1 embodiment.
Figure 11 is a flowchart showing an operation for detecting whether a shortage of water state or not in the embodiment.
Figure 12 is a flowchart showing operation processing to be executed when an operating switch is turned on.
Figure 13 is a flowchart showing an operation when an "interrupt 1" caused in response to the operating switch being turned off occurs in this embodiment.
Figure 14 is a flowchart showing an operation when an "interrupt 2" caused in response to a shortage of water state being detected occurs in this embodiment.
Figure 15 is a flowchart showing an operation when an "interrupt 3" caused in response to a user operating a change-over switch during a normal operation occurs in this embodiment.

### BEST MODE FOR PRACTICING THE INVENTION

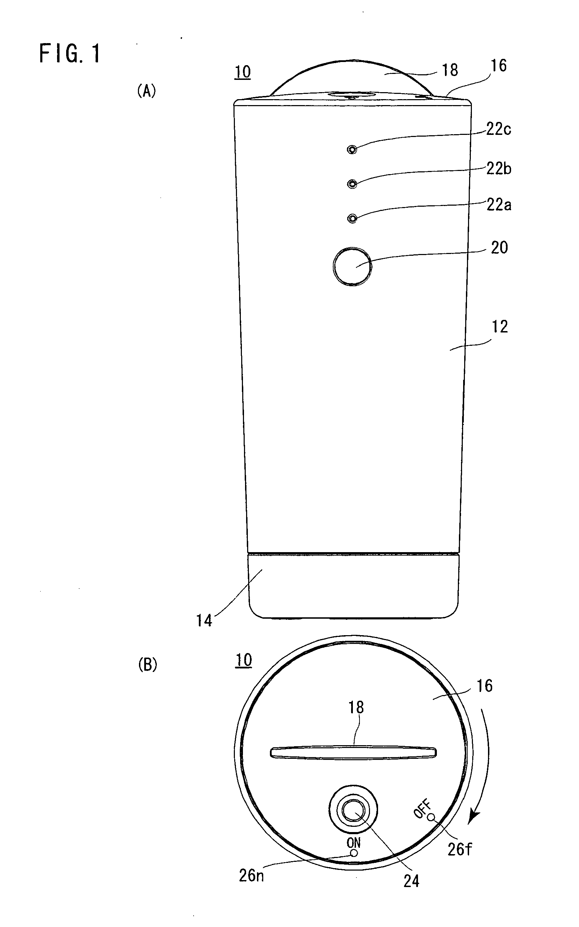

Referring to Figure 1, an atomizing apparatus 10 in this embodiment is basically operated by taking a rechargeable battery (not shown) as a power source, and is formed into a cylindrical shape being smaller in diameter toward the downward direction in the order of 10 cm in diameter and 20 cm in height as a whole. If the atomizing apparatus 10 is intended to be used in the car, the diameter is selected to fit in a hole-like cup holder formed on an inner surface of the doors of the car, or an annular cup holder attached below a dashboard (both not shown). Here, the atomizing apparatus 10 in this embodiment is portable, and can be freely set not only in the car but also on a desk or in a toilet.

Furthermore, it is to be understood that almost all of the components of the atomizing apparatus 10 of this embodiment to be explained below are plastic molded products unless otherwise annotated.

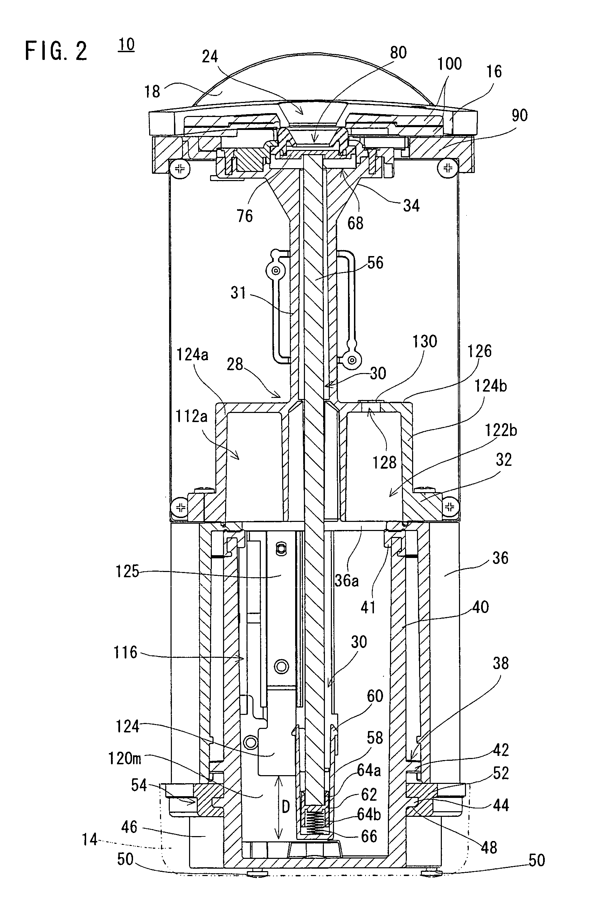

The atomizing apparatus 10 includes a housing 12 deciding the above-described diameter and having a height that extends the entire length of the apparatus. The housing 12 is opened at both upper and lower ends, and detachably attached with a waterproof cover 14 to cover a tank 40 (Figure 2) for water reservoir at a lower end. The waterproof cover 14 is integrated with the tank, and the waterproof cover 14 is rotated to thereby be attached to and detached from the housing 12 together with the tank. In the tank covered with the waterproof cover 14, liquid such as water (specifically tap water is suitable from the reason described later) is retained.

On an upper end of the housing 12, a lid 16 is rotatably attached. The lid 16 is provided with a knob 18 on its top surface, and the lid 16 can be rotated or turned with an operational feeling of click between an on-position and an off-position with the knob 18 held. That is, by rotating the lid 16, it is possible to control on-off actions of the atomizing apparatus 10.

On a front surface of the housing 12, a switch 20 is provided, and the switch 20 is a push button switch for changing an amount of atomizing, that is, the strength of humidification, and every time that the switch 20 is pushed, it is possible to alternately set a "strong" and a "weak". Here, in this embodiment, if the "strong" is set, mist is continuously diffused, but if the "weak" is set, mist is intermittently diffused, such as a one-second-ON and a 0.5-second-OFF. However, the setting between the strong and the weak is not restricted to that of this embodiment, and can take an arbitrary specification.

Above the switch 20 on the front surface of the housing 12, three LEDs 22a, 22b and 22c are arranged in a vertical direction to be aligned with the switch 20. The lowermost LED 22a is for displaying whether or not the aforementioned battery is charged, and lit when charged. The LEDs 22b and 22c are lit to individually display the "strong" and "weak" switched by the switch 20. Here, the LEDs 22b and 22c are two-color LEDs, for example, and also function as a shortage of water state displaying (notifying) means as described later.

The lid 16 is formed with an emission opening 24 for emitting mist as well understood from a plan view shown in Figure 1 (B). An "ON" display 26n is formed at the position aligned with the emission opening 24, and an "OFF" display is set at a position circumferentially spaced apart therefrom. When the lid 16 is rotated to a position (on-position) such that the "ON" display 26n is aligned with the switch 20, an atomizing operation is performed as described above.

Figure 2 is an illustrative view, partially broken away, of the inside of the atomizing apparatus 10 with the housing 12 shown in Figure 1 (A) removed. Inside the housing 12, a frame 28 having approximately the same height as the length of an upper half of the housing 12 is provided. The frame 28 has a side wall 31 for forming a hollow portion 30 below the emission opening 24 in Figure 1(B), and at a lower end of the frame 28, that is, at an approximately middle of the height of the atomizing apparatus 10, an attaching portion 32 is formed, and at an upper end of the frame, a placing portion 34 is formed.

To a bottom surface of the attaching portion 32, a cylindrical casing 36 which has a top plate 36a at an upper end and is opened at a lower end, although its front surface is cut out in Figure 2, is attached such that the top plate 36a is intimate contact with a bottom surface of the attaching portion 32 by a screw, for example. The casing 36 is for detachably loading a tank 40 as a water reservoir means, and is formed with a spiral groove 38 on its inner peripheral surface. On an outer peripheral surface of the tank 40, a spiral ridge 42 to threadedly be engaged with the groove 38 is formed.

On the outer periphery of the tank 40, an annular ridge 44 is formed below the aforementioned spiral ridge 42. The annular ridge 44 is engaged with an annular groove 48 formed on an inner peripheral surface of a packing 46 taking a shape of a cylinder with open ends and made of elastic material such as synthetic rubber. Although simply illustrated by a two-dotted line in Figure 2, the waterproof cover 14 shown in Figure 1 is secured to the outside of the packing 46 by a screw 50.

On an upper end of the packing 46, a flange 52 protruded outwardly as a sealing portion is formed, and the flange 52 is relatively thick in a radius direction, and on an inner surface of the flange 52, the above-described annular groove 48 is formed. On an outer surface of the sealing portion 52, a step 54 is formed. The step 54 is engaged with a step (not illustrated) formed on an upper inner surface of the waterproof cover 14, so that the waterproof cover 14 is securely attached to the packing 46.

Thus, the waterproof cover 14 is integrated with the packing 46, and the packing 46 and the tank 40 are integrated by the engaging portions, that is, the annular ridge 44 and the annular groove 48, so that the waterproof cover 14 and the tank 40 are eventually integrated. Accordingly, by rotating the waterproof cover 14, it is possible to rotate the tank 40 together therewith. Thus, by rotating the waterproof cover 14 counterclockwise (seen from a bottom surface of the atomizing apparatus 10), for example, the spiral ridge 42 can be released from the spiral groove 38 to thereby detach the tank 40 from the casing 36. On the contrary, by rotating the waterproof cover 14 clockwise (seen from the bottom surface of the atomizing apparatus 10), the spiral ridge 42 can be screwed into the spiral groove 38 to thereby attach the tank 40 to the casing 36. Accordingly, when water in the tank 40 is lost to be placed in a water-shortage state, the waterproof cover 14 may be rotated to detach the tank 40 from the casing 36, and after water (tap water) is replenished in the tank, the tank 40 is screwed into the casing 36 again with the waterproof cover 14 held. Thus, the tank 40 is in a form of a closed-bottom cylindrical cup, and the tank 40 with the tap water replenished is attached to the casing 36.

The tank 40 is covered with a ring-shaped packing 41 being made of elastic body like synthetic rubber at an upper end of a side wall. When the tank 40 is screwed into the casing 36, the packing 41 is strongly pressed against the top plate 36a of the casing 36. Thus, a sealing means for the tank 40 is made up of the packing 41 and the top plate 36a.

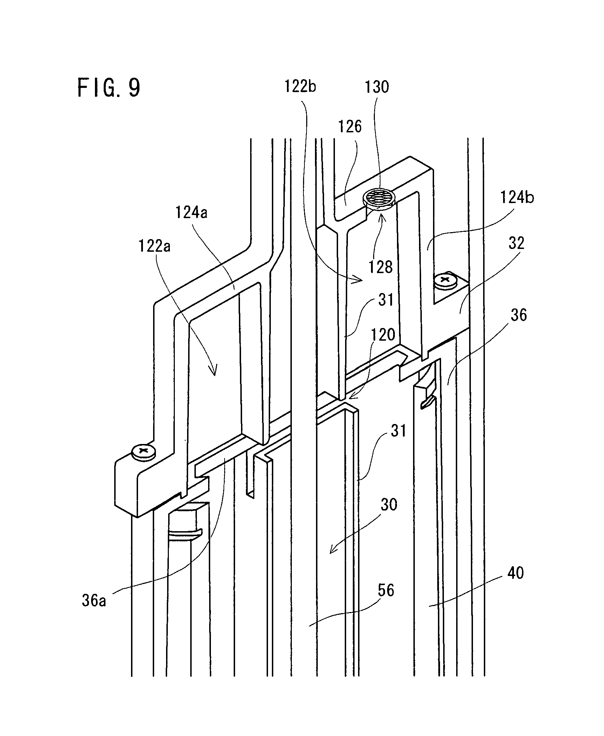

The hollow portion 30 of the frame 28, that is, the side wall 31 is also formed below the attaching portion 32 as especially understood from Figure 9, and thus, the hollow portion 30 is formed so as to extend from the upper end opening of the tank 40 toward the inside of the tank 40. A window (not illustrated) is formed at a part of the side wall 31 of the hollow portion below the attaching portion 32 (Figure 9). Accordingly, the hollow portion 30 provides a communication with the water (not illustrated) in the tank 40 through the window. That is, the water in the tank 40 permeates into the hollow portion 30.

The hollow portion 30 contains a feedwater rod 56 being made of felt (laminated and waulked fibers), for example, having a length approximately equal to the entire length of the height of the hollow portion. The felted material forming the feedwater rod 56 is a product made of laminated fibers, and thus exerts capillary function with respect to liquid. The water in the tank 40 permeates into the hollow portion 30 while the feedwater rod 56 is contained in the hollow portion 30, so that the water is supplied toward the upper potion of the feedwater rod 56 by the capillary function of the feedwater rod 56.

When holding structure of the feedwater rod 56 is described in detail, a closed-bottom cylindrical cap 58 is detachably attached to the lower end of the side wall of the hollow portion 30 such that an upper end of a wedge-shaped engaging portion 60 is engaged with an engaged portion (not illustrated) formed on the side wall of the hollow portion 30. Here, the side wall of the hollow portion to be formed with the engaged portion is not illustrated in Figure 2, but the side wall 31 is well illustrated in Figure 9.

Inside the cap 58, a holder 62 whose vertical cross-section is an "H" letter and having concave portions up and down is housed. An upper concave portion 64a of the holder 62 receives the lower end of the above-described feedwater rod 56, and the lower concave portion 64b contains a coil spring 66. The lower end of the coil spring 66 is in contact with the bottom surface of the cap 58, so that by the elastic repulsion force of the coil spring 66, the holder 62 is forced upwardly. Thus, the feedwater rod 56 whose lower end is supported by the holder 62 is forced upwardly.

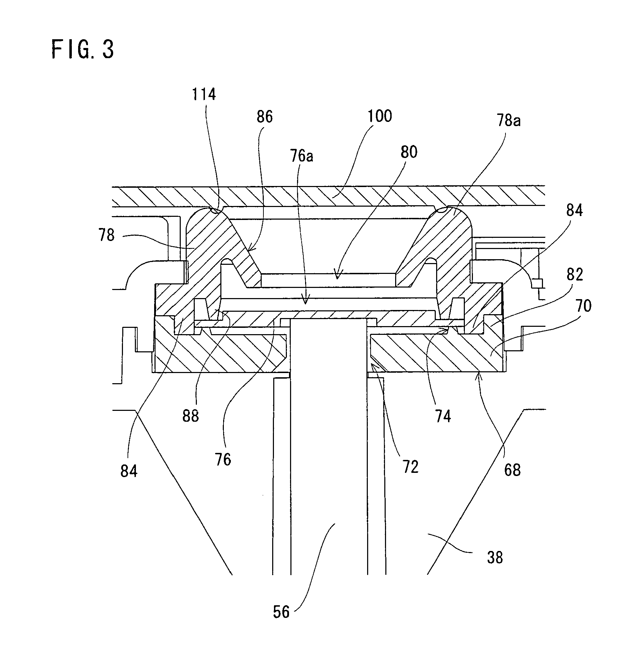

At the upper end of the frame 28, the placing portion 34 is formed as described above. A shape of the outer surface of the placing portion 34 is a funnel shape, and on the upper surface, a recessed portion 68 is formed. In the recessed portion 68, a donut-shaped lower packing 70 being made of elastic material such as synthetic rubber well seen from Figure 3 is placed. The aforementioned upper end of the feedwater rod 56 is presented to an opening 72 of the lower packing 70. Here, the position of the opening 72 corresponds to the position of the emission opening 24, shown in Figure 1 (B).

On the upper surface of the lower packing 70, a circumferential sealing portion 74 whose cross-section is acute triangular and whose plan view is donut shape is formed, and on the sealing portion 74, a disk-like ultrasonic wave vibrator (may be simply referred to as "vibrator") 76 as one example of a mist generator is placed. The vibrator 76 generates high frequency (ultrasonic wave) vibrations by applying a voltage to piezoelectric ceramics, and has a vibrating portion 76a at the center thereof. Since the detail of the vibrator of such a kind has been well known, a further explanation is omitted here.

The placing portion 68 is formed on the hollow portion 30, and at the placing portion 68, the vibrator 76 is placed on the lower packing 70, so that the vibrator 76 is positioned on the hollow portion 30, that is, the feedwater rod 56.

An upper packing 78 having an opening 80 formed at a position overlaid with the position of the opening 72 of the lower packing 70 and being made of elastic material such as synthetic rubber is integrally assembled with the lower packing 70 through adhesion by an adhesive. That is, at an upper surface of an outer periphery of the lower packing 70, an annular protrusion 82 formed to be projected upward is formed, and an outer peripheral surface of an annular protrusion 84 formed to be projected downward from a bottom surface of the outer periphery of the upper packing 78 is closely engaged with an inside peripheral surface of the protrusion 82, and a ridge formed on an upper surface of the protrusion 82 and a groove formed on a bottom surface of the protrusion 84 are engaged with each other, so that the lower packing 70 and the upper packing 78 are integrated tightly.

The opening 80 of the upper packing 78 is defined by a funnel-shape tilted portion 86 which is tilted inward as it going down, and the center of the tilted portion 86 is formed as the opening 80.

Between the aforementioned tilted portion 86 of the upper packing 78 and the aforementioned protrusion 84, a sealing portion 88 whose cross-section is triangular and whose plan view is annular is formed to be projected downward. As described above, the vibrator 76 is placed on the sealing portion 74 of the lower packing 70, and thus, the top of the sealing portion 74 is brought into engagement with a bottom surface of the vibrator 76. On the other hand, as described above, the top of the sealing portion 88 of the upper packing 78 projected downward is brought into contact with a top surface of the vibrator 76 at a position more inward than the position where the sealing portion 74 is brought into contact with the bottom surface. That is, the vibrator 76 is securely retained on the placing portion 68 by being sandwiched by the sealing portions 88 and 74.

As described above, since the feedwater rod 56 is forcedly upward by the coil spring 66 (Figure 2), the upper end of the feedwater rod 56 is securely brought into contact with the central bottom surface of the vibrator 76 through the opening 72 of the lower packing 70. The feedwater rod 56 gradually and continuously sucks up water in the tank 40 because it is made of a felted material, for example as described above. The water that reaches the upper end of the feedwater rod 56 is converted into mist by the vibrator 76, and the mist is emitted (atomized) through the opening 80 of the upper packing 78 and the emitting opening 24 of the lid 16 to exert a humidifying function. Here, the emitting opening 24 is subject to be positionaly overlapped with the opening 80.

As shown in Figure 3 and explained above, the vibrating portion 76a of the vibrator 76 is surrounded by the upturned sealing portion 74 of the lower packing 70 on the bottom surface of the vibrator 76, and the vibrating portion 76a is surrounded by the downturned sealing portion 88 of the upper packing 78 on the upper surface, so that it is possible to surely prevent leakage of the water on the top and the bottom surfaces of the vibrator 76 and undesired emission of mist from occurring.

For example, assuming that the force pushing up the feedwater rod 56 by the coil spring 66 is F1, and the force applied from the sealing portion 88 of the upper packing 78 to retain the vibrator 76 against the lower packing 70 is F2, each of forces is set so as to satisfy a relation of F2>F1, whereby it is possible to surely retain the vibrator 76 against the lower packing 70 and prevent water leakage from occurring.

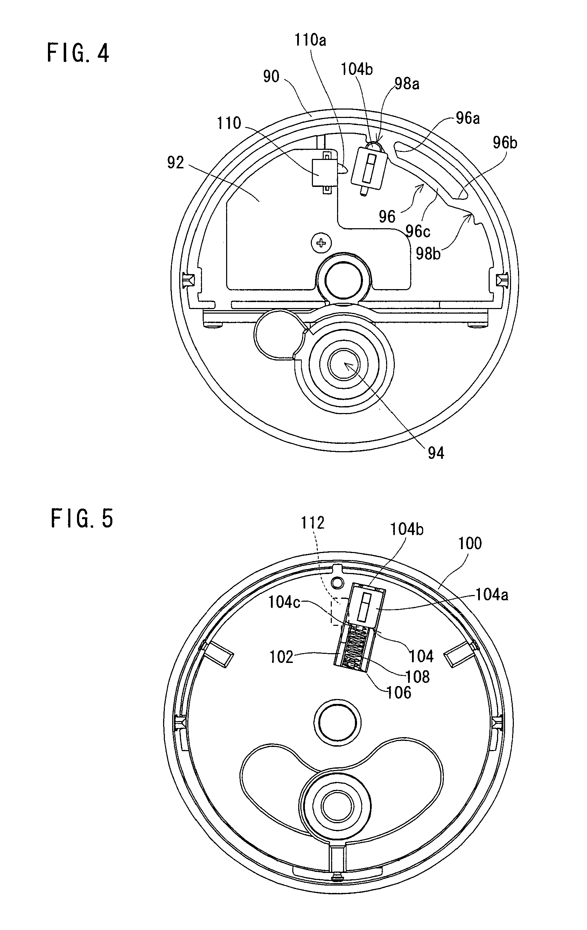

At the upper end of the frame 28, a fixing plate 90 shown in a plan view in Figure 4 is secured. On the fixing plate 90, a circuit board 92 is attached and on the circuit board 92, electronic components for making up of the electric circuit is mounted and wired in a predetermined manner, but these are not illustrated in Figure 4 in order to make the drawings simple.

On the fixing plate 90, an opening 94 is formed at a position overlaid on the aforementioned opening 80 (Figure 3). Accordingly, mist is finally emitted or atomized to the outside through the opening 80, the opening 94 and the emitting opening 24.

On the upper surface of the fixing plate 90, a rotation guide 96 for implementing a step-by-step rotation (click stop) the lid 16 as described is formed. The guide 96 is a communication member, formed in a predetermined circumferential position closer to an outer periphery of the fixing plate 90, includes slanting sides 96a and 96b on both sides and a connecting side 96c sandwiched between the slanted sides 96a, 96b. The connecting side 96c is curved such that the both ends are at slightly inward positions and the center is at an outward position like the outer peripheral edge of the fixing plate 90. In addition, outside root ends (outer periphery sides) of the slanting sides 96a and 96b, recessed portions 98a and 98b are formed. Conversely, the recessed portions 98a and 98b are communicated by the guide 96.

As well understood from Figure 2, on the bottom surface of the lid 16, a two-layer lower surface plate 100 is assembled so as to be integrated with the lid 16. The lower surface plate 100 may be originally integrally produced with the lid 16, but this is separately prepared from the lid 16 for facilitating the injection molding. In this sense, the lower surface plate 100 can be regarded as a part of the lid 16.

As shown in Figure 5, on the lower surface plate 100, that is, the bottom surface of the lid 16, a concave portion 102 whose shape is approximately a rectangle and whose longitudinal direction is along a radial direction from the center is formed. On the concave portion 102, a following member 104 is installed. The following member 104 includes a main body 104a, a protruded portion 104b whose tip end is circular and which is provided at a leading end of the main body 104a, and a lug 104c which is provided at a trailing end of the main body 104a, and between the lug 104c and a lug 106 formed on a bottom side of the concave portion 102, a coil spring 108 is interposed. Accordingly, the main body 104a of the following member 104, that is, the protruded portion 104b is always elastically energized by the coil spring 108 forward. Thus, the protruded portion 104b is pressed against the above-described rotation guide 96 provided in front thereof.

The fixing plate 90 shown in Figure 4 and the lower surface plate 100 shown in Figure 5 are placed in a corresponding positional relation, and this is equivalent to a situation in which the "ON" display 26n on the lid 16 shown in Figure 1 (B) is placed in the on-position, that is, placed to be aligned with the switch 20 shown in Figure 1(A). At this time, the leading end protruded portion 104b of the following member 104 is engaged in the one recessed portion 98a formed outside the both ends of the guide 96. The main body 104a of the following member 104 is in a state pressed forward by the coil spring 108, so that the lid 16 is retained at this position.

On the other hand, on the circuit board 92 (Figure 4), a limit switch 110 functioning as a operating switch is attached, and an actuator 110a of the limit switch 110 is projected from the circuit board 92 toward the guide 96.

An acting portion 112 represented by dotted line in Figure 5 is a protrusion projected downward from the bottom surface of the lower surface plate 100, and acts on the actuator 110a of the limit switch 110 at the on-position shown in Figure 4 and Figure 5 to push it. Accordingly, at this on-position, the limit switch 110 is placed in an on-state, so that the atomizing apparatus 10 is placed in an activating state, that is, an operating state as described later.

When from the on-state, the lid 16 is rotated with the knob 18 held by a certain degree or more force in an arrow direction shown in Figure 1 (B), the protruded portion 104b of the following member 104 is disengaged from the recessed portion 98a and moves along the slanting side 96a, abutting the inner surface of the slanting side 96a. Since in this situation, the pressing of the actuator 110a of the limit switch 110 by the protrusion of the lid 16, that is, the acting portion 112 is released, and the limit switch 110 becomes the off-state, so that the operation of the atomizing apparatus 10 is stopped.

Since the slanting side 96a of the guide 96 is inclined inwardly, the protruded portion 104b of the following member 104, that is, the main body 104a is pressed backward against the elastic force of the coil spring 108, and in accordance with a continual rotation of the lid 16, the protruded portion 104b of the following member 104 moves with reposing on the connecting side 96c of the guide 96. At this time, the coil spring 108 is in the most compressed state.

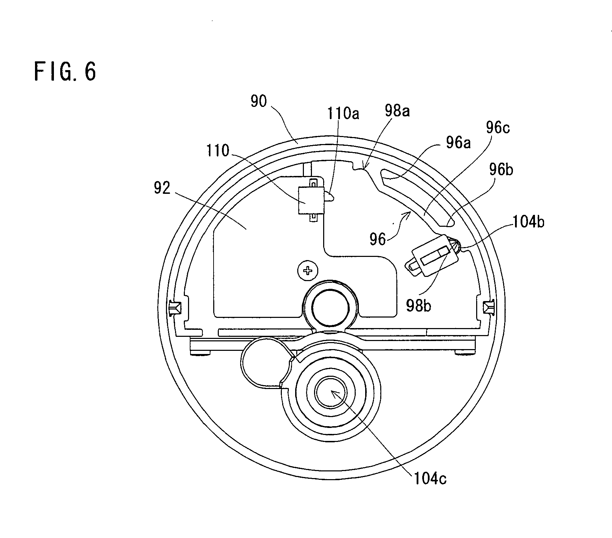

When the lid 16 is further rotated or continuously rotated, the protruded portion 104b of the following member 104 moves along the other slanting side 96b of the guide 96. Accordingly, the compression of the coil spring 108 is gradually released to press forward the main body 104 of the following member 104. In accordance with the continuation of the rotation, the leading end protruded portion 104b of the following member 104 is engaged with the other recessed portion 98b, snaps down there by the pressure of the coil spring 108, and then, the rotation of the lid 16 is stopped, and the lid 16 is retained at this position. The situation is an off-state in which the "off" display 26f of the lid 16 is aligned with the switch 20. The positional relation between the protruded portion 104b of the following member 104 in the off-state and the guide 96 is shown in Figure 6.

Thus, the lid 16 is rotated in a step-by-step manner and switched between ON and OFF by the spring 108, the following member 104, that is, the protruded portion 104b, and the guide 96 including the recessed portions 98a, 98b functioning as a follower. Then, on both states, the on-state and the off-state, the protrusion is engaged with each of the recessed portions, so that it is possible to securely retain the on-state and the off-state. Accordingly, even if the atomizing apparatus 10 of this embodiment is held in a cup holder (not illustrated) in a car, an undesirable changeover between the on-state and the off-state due to vibrations transmitted from the car can be prevented.

Here, as to the recessed portions 98a, 98b and the protruded portion 104b, on the contrary to the embodiment shown in Figure 4 and Figure 5, the recessed portions 98a, 98b are formed on the lid 16, that is, the lower surface plate 100, and the protruded portion 104b to be engaged therewith may be formed on the fixing plate 90.

Returning to Figure 3, the force F2 that the aforementioned upper packing 78 applies to the vibrator 76 is maintained by the lower surface plate 100 of the lid 16. That is, at a position corresponding to the top portion 78a of the upper packing 78 on the bottom surface of the lower surface plate 100, a downward annular ridge 114 is formed. Then, when the annular ridge 114 is pressed against the top portion 78a of the upper packing 78 by the lid 16, the aforementioned F2 is applied to the upper packing 78, and a water seal between the upper packing 78 and the lower surface plate 100, that is, the lid 16 is implemented, capable of efficiently preventing water leakage therefrom from occurring. Here, as shown in Figure 3, the seal of the top portion 78a of the upper packing 78 by the ridge of the lower surface plate 100 may be implemented only when the lid 16 is rotated to the on-position. When the lid 16 is placed in the off-position, the operation is in a shut-down state, and the mist is not generated, so that the mist never enters the housing 12 (Figure 1) even if the opening 80 is not sealed. In addition, the pressure of the lid 16 against the lower surface plate 100 is given by a holding means (not illustrated) for securely holding the lid 16 and an integrating means (not illustrated) such as a screw for integrating the lid 16 and the lower surface plate 100.

The atomizing apparatus 10 shown in Figure 1 embodiment further includes other features, one of which is that by performing electrolysis processing on water including chloride ion like tap water accumulated in the tank 40 to produce electrolyzed water, and by dissipating the electrolyzed water as mist, this is directed to exert a function as a humidifier and an effect of suppression of viruses (disinfection) as well. That is, by performing electrolysis on the tap water in the tank 40 to generate two kinds of active oxygen for suppressing virus, such as "hypochlorous acid" and "hydroxyl radical", retaining the active oxygen in a high concentration in the water in the tank 40, and emitting it with water as mist, this is directed to exert an effect of disinfection around the atomizing apparatus 10 (http://www.sanyo.co.jp/vw/concept/index.html).

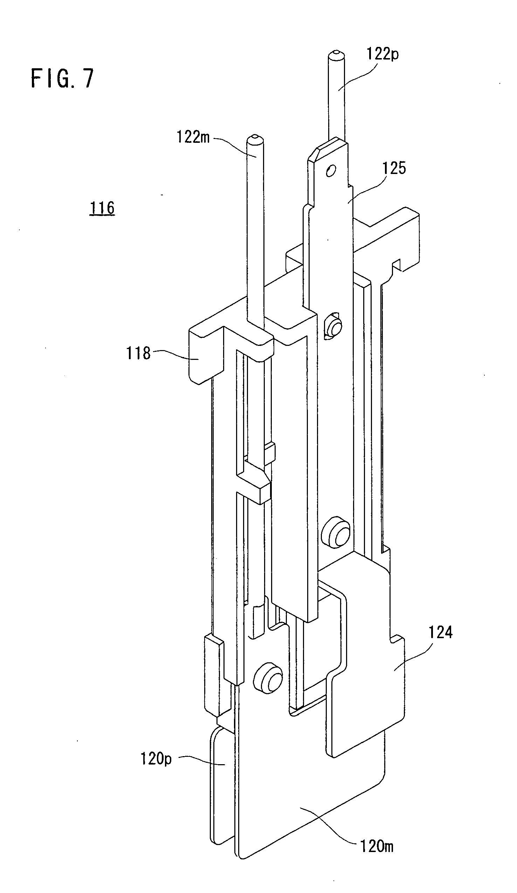

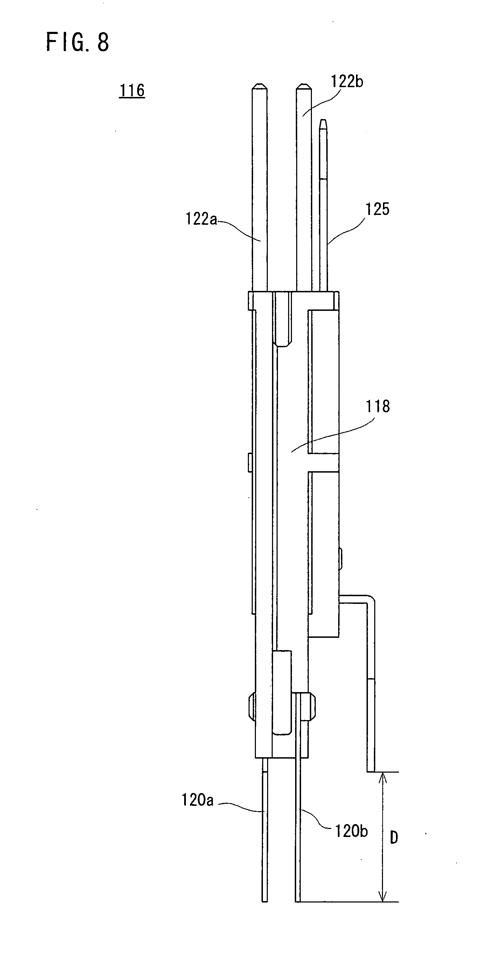

For such electrolysis processing, an electrode unit 116 shown in Figure 7 and Figure 8 is utilized in this embodiment.

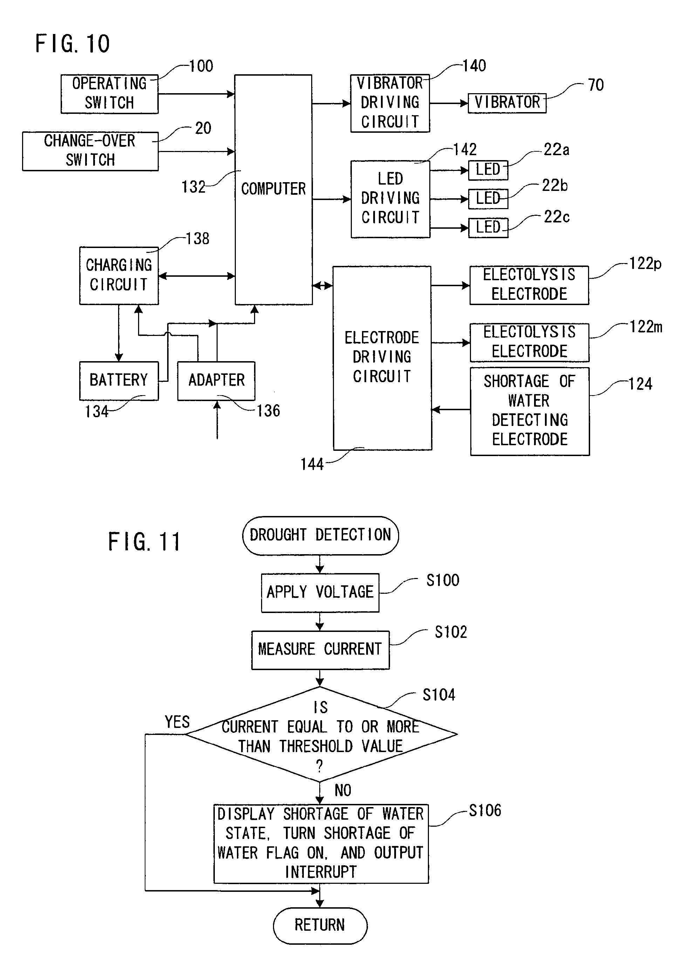

The electrode unit 116 takes approximately the shape of rectangle, and includes a holding plate 118 being formed with an electrode holding portion necessary for holding the electrodes such as grooves and protrusions on both sides. On the one main surface of the holding plate 118, an electrode 120p as a positive electrode is attached by an appropriate means, such as by a screw or by swaging, for example. On the other main surface of the holding plate 118, an electrode 120m as a negative electrode is attached by a similar attaching means. The electrodes 120p and 120m are made of electrically conducting material, such as copper, to be worked into a planar shape, are connected to an electrode driving circuit 144 (Figure 10) described later by round-bar shaped connecting members 122p and 122m, respectively, each being made up of similar electrically conducting material, and receive direct-current power supply from the driving circuit to cause electrolysis.

The electrode unit 116 is attached with a shortage of water detecting electrode 124 that is made of electrically conducting material similar to the above-described electrodes 120p and 120m but separately set, on the other main surface of the holding plate 118 in parallel with the electrode 120m in this embodiment. The third electrode 124 is for detecting whether or not a certain amount of water or more is accumulated in the tank 40, and has a lower end positioned higher than the lower ends of the electrodes 120p and 120m by a distance D as specifically well understood from Figure 8. The shortage of water detecting electrode 124 is connected to the aforementioned electrode driving circuit 144 by a planar connecting member 125.

Such an electrode unit 116 is installed below the bottom surface of the attaching portion 32 of the frame 28 as shown in Figure 2, and in this state as well, the shortage of water detecting electrode 124 is attached in such a position that its lower end is higher than the lower ends of the electrolysis electrodes 120p and 120m by the distance D.

In electrolysis, a DC voltage is applied across the two electrodes 120p and 120m as well known, and if the electrode 124 is under water at that time, a closed circuit is formed as well between the electrode 120p and the shortage of water detecting electrode 124 when the voltage is applied. However, if the shortage of water detecting electrode 124 is not under water, even if a voltage is applied to the electrode 120p, an electric circuit is not formed between the electrodes 120p and 124. Thus, by detecting whether or not a current flows into the shortage of water detecting electrode 124 when a voltage is applied to the electrode 120p, it is possible to detect whether or not a certain amount of water or more remains in the tank 40. A shortage of water detector which detects a shortage of water state within the tank 40 by the current flowing into the shortage of water detecting electrode 124 is a computer 132 (Figure 10).

As clearly understood, the "predetermined amount" is set by the aforementioned distance D, and by setting the distance D at will, it is possible to arbitrarily set the predetermined amount. The state that the water in the tank 40 is below the predetermined amount is called a shortage of water state, and therefore, the electrode 124 is a shortage of water detecting electrode.

In the atomizing apparatus 10 of this embodiment, as described before, the tank 40 is screwed into the casing 36 with the tank 40 with water. At this time, the tank 40 is not entirely filled with water, and has naturally air above the water. If the tank 40 is screwed into the casing 36 in that state, the remaining air is compressed to sometimes make the water overflow from the tank 40.

Hereupon, in the atomizing apparatus of this embodiment, an air passage as shown in Figure 9 is formed to prevent the water from overflowing when the tank 40 is attached.

Figure 9 is illustration for showing the surrounding of the attaching portion 32 of the frame 28 shown in Figure 1, and the casing 36 is mounted to the attaching portion 32, and the tank 40 is installed in the casing 36.

The side wall 31 of the hollow portion 30 for housing the feedwater rod 56 is actually divided up and down by a gap 120 formed due to the top plate 36a of the casing 36 attached to the attaching portion 32 as shown in Figure 9 in detail. The gap 120 is formed on the walls 31 on both sides of the hollow portion 30 which sandwich the feedwater rod 56. In addition, side walls 124a and 124b for defining the spaces 122a and 122b are formed on the attaching portion 32, and on a ceiling 126 of the one side wall 124b, an air hole 128 is formed. The air hole 128 is provided with a filter 130. Although it is roughly drawn for the sake of illustration, a mesh size of the filter 130 is set to such a size that air molecules can be transmitted but water molecules cannot be transmitted.

When the tank 40 is screwed into the casing 36, the air within the tank 40 is gradually compressed, passes through the opening at the upper end of the tank 40, and finally goes through the air hole 128 to outside. At this time, in Figure 9, the air going out from the left side of the opening of the tank 40 flows into the space 122b through the gap 120 of the side wall 31 of the hollow portion 30, and goes out through the air hole 128. In Figure 9, the air going out from the right side of the opening of the tank 40 directly flows into the space 122b and goes out through the air hole 128. That is, in this embodiment, the air in the tank 40 is always communicated with the atmosphere through the passage including the gap 120, the space 122 and the air hole 128, so that a phenomena that a remaining air is compressed when the tank 40 is attached does not occur, capable of preventing the inconvenience of overflowing of the water in the tank 40 occurring.

Since the aforementioned filter 130 is set to the air hole 128, even if the water in the tank 40 overflows in the spaces 122a, 122b due to big shakes of the car, the water never spills through the air hole 128.

An electronic circuit configuration of the atomizing apparatus 10 of such structure is shown in a block diagram shown in Figure 10. Referring to Figure 10, the atomizing apparatus 10 includes the computer 132 for controlling an entire electronic operation of the atomizing apparatus 10, and the computer 132 is supplied with direct-current power from a battery 134 or an adapter 136. Although roughly illustrated in Figure 10, a direct-current power supply from an adapter 136 is also applied to a charging circuit 138. Thus, the battery 134 is charged by the charging circuit 138. Although not illustrated in Figure 2, the battery 134 is loaded at a position hidden in Figure 2 above the attaching portion 32 of the frame 28.

Here, a less current is flown through the computer 132 even in an off-state of the operating switch 110 to make the computer 132 persist a standby state, and also causes the data in the internal memory (not shown) of the computer 132 to be held in the memory.

The computer 132 is connected with the limit switch 110 shown in Figure 4 as an operating switch, and also connected with the changing-over switch 20 (Figure 1).

The computer 132 instructs a vibrator driving circuit 140 to perform on-off control of the vibrator 76. The computer 132 further instructs an LED driving circuit 142 to make on-off control of the LEDs 22a, 22b and 22c.

The computer 132 controls an electrode driving circuit 144. The electrode driving circuit 144 applies a direct voltage for electrolysis across the electrolysis electrodes 120p and 120m. The computer 132 further detects whether or not a current flows into the shortage of water detecting electrode 124 on the basis of a signal from the electrode driving circuit 144.

When a rotation of the lid 16 to the on-position makes the acting portion 112 act on the actuator 110a of the limit switch 110, to thereby turn the limit switch, that is, the operating switch 110 on, the computer 132 instructs the vibrator driving circuit 140 to control the vibrator 76 to an on-state. Thus, an atomizing operation by the atomizing apparatus 10 is performed.

Figure 11 is a flowchart showing an operation allowing the computer 132 to detect whether or not a predetermined amount of water or more is in the tank 40, in other words, whether or not the tank 40 is in a shortage of water state, by the shortage of water detecting electrode 124.

In a first step S100, the computer 132 instructs the electrode driving circuit 144 to apply a direct voltage to the electrolysis electrode 120p.

In a succeeding step S102, the computer 132 measures a current flowing into the shortage of water detecting electrode 124 in the electrode driving circuit 144 with the voltage applied in the step S100.

Then, in a step S104, the computer 132 determines whether or not a current value is equal to or more than a predetermined threshold value. The fact that the predetermined amount or more current flows into the shortage of water detecting electrode 124 means that the shortage of water detecting electrode 124 is under water in the tank 40, so that a predetermined amount or more of water remains in the tank 40. Accordingly, if "YES" is determined in the step S104, that is, if the shortage of water state in the tank 40 is not detected, the shortage of water detecting processing is ended.

If "NO" in the step S104, this means that the shortage of water detecting electrode 124 is not fully soaked in the water of the tank 40, and the tank 40 is placed in the shortage of water state. When it is detected that the tank 40 is placed in the shortage of water state, the computer 132 instructs the LED driving circuit 142 to inform the user of a shortage of water state by turning on the two LEDs 20b and 20c with the red light in a step S106. Then, the computer 132 writes "1" to a shortage of water flag (memory for memorizing whether a shortage of water state or not by "1" or "0".) set to a memory (not illustrated) of the computer 132, and outputs an interrupt ("interrupt 2" described later) based on the shortage of water state detection in the step S106.

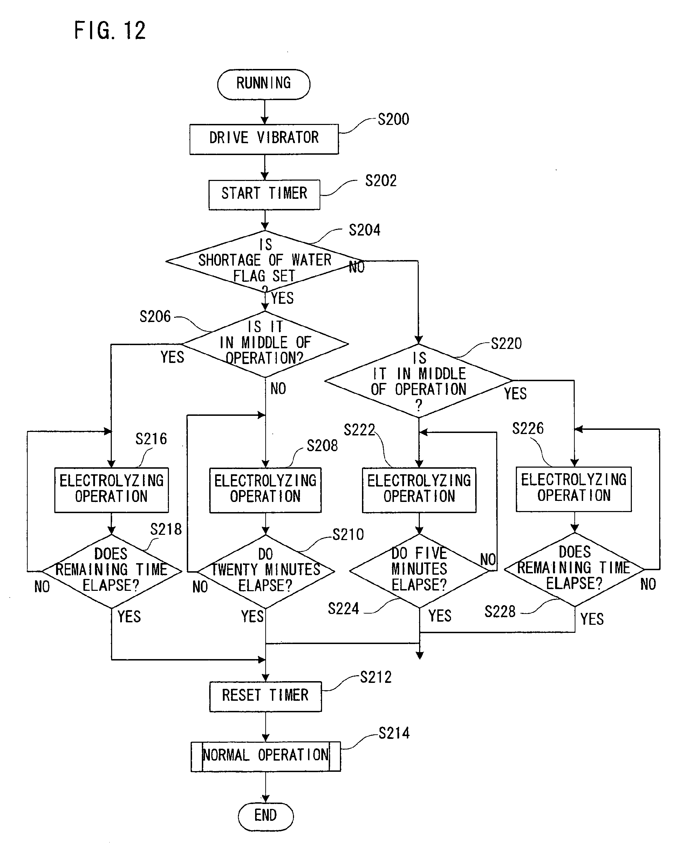

Such shortage of water detecting processing is simultaneously executed "during execution of an initial electrolyzing operation" explained with reference to Figure 12. This is because that a voltage has to be applied to the electrode 120p for shortage of water detection as well as described above, resulting in consumption of the electric power. Accordingly, in this embodiment, this is executed in parallel with the electrolysis processing to thereby reduce electric power consumption. In this sense, the step S100 is the same as an operation of applying a voltage to the electrolysis electrode 120p for the initial electrolyzing operation, and does not require special processing for shortage of water detection.

As in this embodiment, in a case that the water detecting processing is executed during the initial electrolyzing operation in parallel, the program shown in Figure 11 is executed at a plurality number of times, for example, twice during the operation.

The shortage of water detection may be performed at another timing, but in this case, the magnitude of the voltage to be applied to the electrode 120p can be changed.

Referring to Figure 12, Figure 12 is a flowchart showing operation processing to be executed when the user rotates the lid 16 to the on-position to thereby turn the operating switch 110 on.

When detecting that the operating switch 110 is turned on, the computer 132 instructs the vibrator driving circuit 138 to control the vibrator 76 to the-on-state in a step S200. Accordingly, an atomizing operation by the atomizing apparatus 10 is performed.

In a succeeding step S202, the computer 132 starts a timer (not illustrated) set to an appropriate area within the internal memory (not illustrated).

Then, in a step S204, the computer 132 determines whether or not the flag is set, that is, "1" is written to the flag with reference to the shortage of water flag (not illustrated) within the memory. Here, the reason why the computer 132 first determines whether or not the shortage of water flag is set is to determine whether or not the current operation is the first operation after the shortage of water is displayed and the user pours water in the tank 40 in response thereto. As described above, when the shortage of water state is detected in the water detecting processing shown in Figure 11, the computer 132 writes "1" to the shortage of water flag and retains this state, and therefore, by checking the shortage of water flag in the step S204, it is possible to determine whether the first operation after replenishment of the water or not.

The atomizing apparatus 10 of this embodiment is directed to execute a disinfecting function by emitting electrolyzed water mist. Accordingly, if the electrolyzed water concentration of the water in the tank 40 is not equal to or more than a certain amount, it is impossible to expect a disinfection advantage. By merely replenishing tap water in the tank 40, no disinfection substance, such as hypochlorous acid is melted in the tap water. Thus, in this embodiment, in the first operation immediate after the water replenishment, electrolytic processing is executed for a relatively long first time, 20 minutes as an initial electrolytic processing in order to make the electrolyzed water concentration to a certain amount or more in this embodiment.

Here, in a case that it is not the shortage of water state, the initial electrolyzing operation time is set to a relatively short second time, for example, 5 minutes. Normally, the electrolyzed water concentration decreases over the course of hours to days (time period), and therefore, the initial electrolyzing operation is executed every time at the beginning of the operation, although it is relatively short, to thereby maintain the electrolyzed water concentration.

When "YES" is determined in the step S204, it is the first operation after the water replenishment, and therefore, the initial electrolyzing operation during the above-described first time (20 minutes, for example) is executed. Thereupon, in a step S206, it is determined whether or not the operation is started in the middle of the initial electrolyzing operation.

In this embodiment, when the above-described initial electrolyzing operation during the first or the second time is executed, if the lid 16 is rotated to the off-position to turn the operating switch 110 off, it falls short of the initial electrolytic time. Thus, at the beginning of the next operation, the initial electrolyzing operation is continued for the remaining time of the preceding initial electrolyzing operation to thereby retain the electrolyzed water concentration. For example, in a case that the operating switch 110 is turned off when the operation is executed for 10 minutes in spite of the fact that 20 minutes is set as an initial electrolyzing operation time, the remaining time is 10 minutes. In a case that the operating switch 110 is turned off when the operation is executed for 2 minutes in spite of the fact that 5 minutes is set as an initial electrolyzing operation time, the remaining time is 3 minutes. The fact that in response to the application of the voltage at the initial electrolyzing operation, a shortage of water detection operation (Figure 11) is executed is as described above.

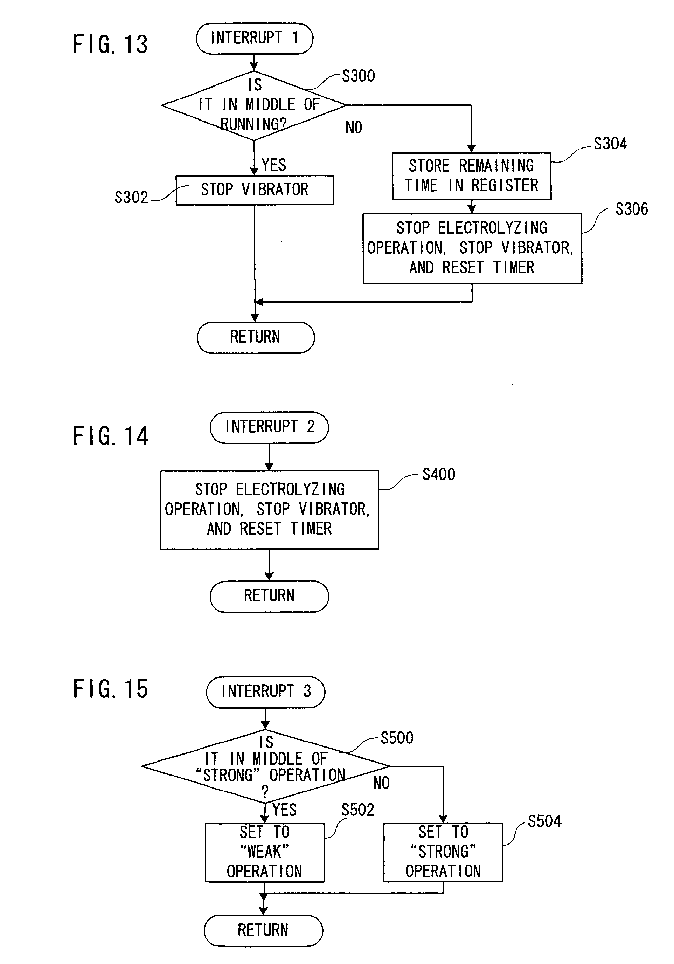

Such remaining time is stored in a remaining time register (not illustrated) of the internal memory of the computer 132 in an interrupt 1 operation resulting from the operating switch 110 being turned off as described later (Figure 13, step S304). Accordingly, in the step S206, by checking whether or not the remaining time is stored in the remaining time register, it is determined whether or not to be in the middle of the operation.

When "NO" is determined in the step S206, that is, when it is not in the middle of the operation, the computer 132 instructs the electrode driving circuit 144 to execute an initial electrolyzing operation by applying a voltage to the electrolysis electrode 120p in a next step S208. The fact that in response to the application of the voltage, a shortage of water detection operation (Figure 11) is executed is as described above.

Thereafter, the initial electrolyzing operation in the step S208 is continuously executed until the computer 132 confirms that 20 minutes elapse with reference to the timer triggered in the step S202 in a step S210. In this sense, the step S210 functions as an initial electrolyzing operation time controller.

Then, when it is determined that 20 minutes elapse in the step S210, the computer 132 resets the timer in a next step S212, and shifts to a humidifying operation in the "normal operation" in a step S214.

When "YES" is determined in the step S206, the computer 132 applies a voltage for electrolysis to the electrolysis electrode 120p to restart the initial electrolyzing operation which was interrupted before similar to the preceding step S208 in a next step S216. The fact that in response to the application of the voltage, a shortage of water detection operation (Figure 11) is executed is as described above. Thereafter, the initial electrolyzing operation restarted in the step S216 is continuously executed until the computer 132 confirms that the remaining time elapses with reference to the timer triggered in the step S202 in a step S218. In this sense, the step S216 also functions as an initial electrolyzing operation time controller.

Here, in order to make the determination in the step S218, the computer 132 determines whether or not the remaining time saved in the remaining time register as described above expires with reference to the timer.

Then, when "YES" is determined in the step S218, the computer 132 executes the next step S212.

When "NO" is determined in the preceding step S204, that is, when the computer 132 determines that the current operation is a operation after the normal operation is stopped (restarted normal operation), the initial electrolyzing operation for the aforementioned second time (5 minutes, for example) is executed. Then, similar to the technique in the step S206, it is determined whether or not to be in the middle of the operation, that is, whether or not the interrupted initial electrolyzing operation is restarted in a next step S220.

When "NO" is determined in the step S220, that is, it is not in the middle of the operation, similar to the step S208 and step S216, the computer 132 instructs the electrode driving circuit 144 to apply a voltage to the electrolysis electrode 120p to thereby execute the initial electrolyzing operation in a next step S222. The fact that in response to the application of the voltage, a shortage of water detection operation (Figure 11) is executed is as described above.

Thereafter, the initial electrolyzing operation in the step S222 is continuously executed until the computer 132 confirms that 5 minutes elapse with reference to the timer triggered in the step S202 in a step S224. In this sense, the step S224 also functions as an initial electrolyzing operation time controller.

Then, when it is determined that 5 minutes elapse in the step S224, the process proceeds to the steps S212 and S214.

When "YES" is determined in the step S220, the computer 132 applies a voltage for electrolysis to the electrolysis electrode 120p to continue the interrupted initial electrolyzing operation similar to the preceding steps S208, S216, S222 in a next step S226. The fact that in response to the application of the voltage, a shortage of water detection operation (Figure 11) is executed is as described above. Thereafter, the initial electrolyzing operation restarted in the step S226 is continuously executed until the computer 132 confirms that the remaining time elapses with reference to the timer triggered in the step S202 in a step S228. In this sense, the step S226 also functions as an initial electrolyzing operation time controller.

Here, in order to make the determination in the step S228, the computer 132 is only necessary to determine whether or not the remaining time held in the remaining time register described above expires with reference to the timer.

Figure 13 is a flowchart showing an operation of the computer 132 when "the interrupt 1" occurs in response to the operating switch 110 being turned off when the user rotates the lid 16 to set to the off-position.

When the interrupt 1 event occurs, the computer 132 first determines whether or not to be in the middle of the normal operation in a step S300. That is, in the step S300, it is determined whether to be in the middle of the initial electrolyzing operation in the step S208, S216, S222, S226 or to be in the middle of the normal operation in the step S214, in Figure 12. As a detection method, it is conceivable that whether or not an electrode driving instruction is output to the electrode driving circuit 144 at that time. The fact that the electrode driving instruction is output means that any one of the steps S208, S216, S222, and S226 in Figure 12 is executed.

When "YES" is determined in the step S300, the computer 132 instructs the vibrator driving circuit 140 to stop driving the vibrator 76 in a next step S302, and then the process is returned.

When "NO" is determined in the step S300, because of the stop of the operation during the initial electrolyzing operation, that is, the interrupt of the initial electrolyzing operation, the computer 132 stores a remaining time in the remaining time register (not illustrated) as described above in a succeeding step S306. In accordance therewith, in the step S306, the computer 132 instructs the electrode driving circuit 144 to stop applying the voltage to the electrolysis electrode 120p, instructs the vibrator driving circuit 140 to stop driving the vibrator 76, further resets the timer (step S202), and then returns the process.

Figure 14 is a flowchart showing an operation of the computer 132 when "the interrupt 2" occurs in response to the shortage of water state being detected during the initial electrolyzing operation.

When the interrupt 2 event occurs, the computer 132 instructs the electrode driving circuit 144 to stop applying a voltage to the electrolysis electrode 120p, instructs the vibrator driving circuit 140 to stop driving the vibrator 76, further resets the timer described in the step S202 in a step S400, and returns the process.

Figure 15 is a flowchart showing an operation of the computer 132 when the "interrupt 3" occurs in response to the user operating the changeover switch 20 (Figure 1, Figure 10) during the normal operation in the step S214 (Figure 12).

When the interrupt 3 event occurs, the computer 132 first determines whether or not to be in the middle of a "strong" operation in a step S500. That is, in the step S500, whether a strong operation mode for continuously emitting the mist or a weak operation mode for intermittently emitting mist is determined with reference to the instruction output to the LED driving circuit 142 in the step S214 shown in Figure 12. That is, when the LED 22b lights up, the "strong operation", and when the LED 22c lights up, the "weak operation".

When "YES" is determined in the step S500, the computer 132 sets the operation mode to the "weak operation". Example is that an operation time and a stop time are set to an intermittent timer (not illustrated). When "NO" is determined in the step S500, the computer 132 sets the operation mode to the "strong operation" by resetting the set time of the intermittent timer and so forth.

Here, although not explained above, the charge of the battery 134 (Figure 10) is constantly executed when the remaining amount of the battery is lowered and the direct voltage is supplied by the adapter 136 (Figure 10), even if the operating switch 110 is turned off, or even if the shortage of water state is detected.

In the above-described embodiment, by using one shortage of water detecting electrode 124, a shortage of water state is detected. However, by using a plurality of water detecting electrodes (shortage of water detecting electrodes) other than the electrolysis electrodes, and by setting the level of each electrode stepwise (more specifically, one being equivalent to the distance D between the lower edge of each water detecting electrode and the lower edge of the electrolysis electrode (Figure 8)), the amount of the water in the tank 40 may be determined stepwise. In such a case, the water amount flag is set in correspondence with the detected amount of water, and depending on the water amount flag, the initial electrolyzing operation time is variably set, and whereby it becomes possible to generate electrolyzed water with a more optimal concentration.

Although the present invention has been described and illustrated in detail, it is clearly understood that the same is by way of illustration and example only and is not to be taken by way of limitation, the spirit and scope of the present invention being limited only by the terms of the appended claims.

## Claims

1. An atomizing apparatus, comprising:
a water storage which stores water including chlorine ion;
an electrolyzer which electrolyzes the water stored in said water storage by a pair of electrolysis electrodes into electrolyzed water;
a mist generator which generates mist of the electrolyzed water in said water storage;
a diffuser which diffuses the mist generated by said mist generator;
a shortage of water detector which detects a shortage of water state when the water in said water storage is below a predetermined amount;
a memory which memorizes the shortage of water state when said shortage of water state is detected by said shortage of water detector;
a determiner which determines whether or not said shortage of water state is stored in said memory at a start of an operation; and
an initial operation time controller which sets a time of an initial electrolyzing operation by said electrolyzer to a different time depending on a determination result by said determiner.

2. An atomizing apparatus according to claim 1, wherein said initial operation time controller sets a first time for said initial electrolyzing operation when said determiner determines that said shortage of water state is memorized in said memory, and sets a second time shorter than said first time when said determiner does not determine that said shortage of water state is memorized in said memory.

3. An atomizing apparatus according to any one of claim 2, further comprising a time storage which stores a remaining time of said initial electrolyzing operation when a stop of the operation is instructed during said initial electrolyzing operation, wherein
said initial operation time controller sets said remaining time as said initial electrolyzing operation time.

4. An atomizing apparatus according to any one of claims 1 to 3, wherein said shortage of water detector includes another shortage of water detecting electrode than said pair of electrolysis electrodes, and detects whether the shortage of water state or not depending on whether or not a predetermined amount of current or more flows through said shortage of water detecting electrode during said initial electrolyzing operation.
